# EUROPEAN PATENT APPLICATION

(11) **EP 3 167 867 A1**
(43) Date of publication of application: **17.05.2017**
(21) Application number: 15194816.3
(22) Date of filing: 16.11.2015
(51) Int. Cl.: A61J 3/07, C09J 5/00

(54) **TAMPERPROOF DOSAGE FORM**

(71) Applicant: Capsugel Belgium NV, 2880 Bornem (BE)
(72) Inventor: Huysmans, Tom, 9100 Sint-Niklaas (BE); Dierckx, Tarryn, 2100 Deurne (BE); Vanquickenborne, Stefaan Jaak, 2820 Rijmenam (BE)
(74) Representative: De Clercq & Partners

(57) **Abstract**

A tamperproof dosage form for oral administration comprising a cap and a body each comprising an outer surface and an inner surface, the cap and body being arranged to telescopically engage with each other such that an overlap region is formed between a portion of the outer surface of the body and a portion of the inner surface of the cap, said overlap region comprising an overlap length extending parallel to a centerline of said dosage form, wherein an adhesive substance is comprised throughout substantially the entire overlap length bonding said cap to said body.

## Description

### FIELD

The present disclosure relates to tamperproof ingestible dosage form articles suitable for the delivery of one or more medicaments or other active materials. More particularly, the dosage form articles are suitable for ingestion by a subject, preferably the subject being selected from humans or animals. Most preferably, the present dosage forms are for use in dry-powder-inhalation applications whereby a dosage form is typically inserted into an inhalation device.

### BACKGROUND

Capsule technology continues to be subject to development and improvements. In its basic form, standard containers for pharmaceuticals or other powdered, granular or liquid substances (generally referred to as telescope-type or two-piece capsules) include a tubular-shaped and/or cylindrically-shaped first part, namely a cap part, which is closed on one end and open on the other opposite end. A tightly fitting second part of similar shape, namely the body part, is of smaller diameter than the cap part and is typically telescopically engaged therein to form the overall dosage form or two-piece capsule. Similar capsule technology may be used to generate multi-compartment capsules.

Sealing of capsules of the above type has been implemented mainly to allow storing of liquids within such capsules. Such sealing techniques include for example using organic solvent based sealing fluids incorporated in a specific portion of the overlap region, typically proximal to the opening of the cap, between capsule shells, as exemplified in US2007/0184077. Although such have been shown to provide excellent resistance to leaking of the liquid content, it may not be as effective for solid or powder filled capsules. Moreover, such techniques typically involve the use of organic solvents which should be desirably avoided.

Alternative sealing techniques use banding techniques to apply a layer of sealant on the outside surface of the capsule along the entire seam of the joined cap and body parts, such is exemplified in US4,756,902 wherein the juncture of the cap and body is sealed with a sealing fluid containing an alcohol-water solution maintained at a temperature of from 40 to 100 degC to form a liquid seal and continuous surface capsule followed by a gelatin band to gird the capsule in the area of the liquid seal. Another example is provided in US3,071,513 wherein the sealing fluid used comprises a dispersion of an airdrying hydrophilic, film-forming polymer in an organic solvent. The application of the sealing fluid is by dipping the capsules. Such techniques however suffer from similar drawbacks.

As an alternative to the above, sealing by applying a sealant between the inner side of the cap and body along the ridge of the body is for example described in US4,196,564. However, again such does not provide tamperproofness to the capsules, particularly when torsion is applied as well as requiring the body of the capsule to be over-filled.

It is therefore desirable to provide a new tamperproof capsule and process of making that overcomes the drawbacks in the known techniques.

It is also desirable to provide a new adhesive composition that is effective in providing tamperproof capsules of a wide range of materials including gelatin and celluloses.

### SUMMARY

In a first aspect, a tamperproof dosage form for oral administration comprising: a cap and a body each comprising an outer surface and an inner surface, the cap and body being arranged to telescopically engage with each other such that an overlap region is formed between a portion of the outer surface of the body and a portion of the inner surface of the cap, said overlap region comprising an overlap length extending parallel to a centerline of said dosage form, wherein an adhesive substance is comprised throughout substantially the entire overlap length bonding said cap to said body.

In a second aspect, an adhesive composition for bonding one or more segments selected from a cap and body of a dosage form to render said dosage from tamperproof, the adhesive composition being an aqueous composition free of organic solvents and comprising one or more adhesive enhancing agents and water.

In a third aspect, a process of making tamperproof dosage forms.

In a fourth aspect, the use of an adhesive composition for tamperproofing dosage form articles.

### BRIEF DESCIPTION OF THE DRAWINGS

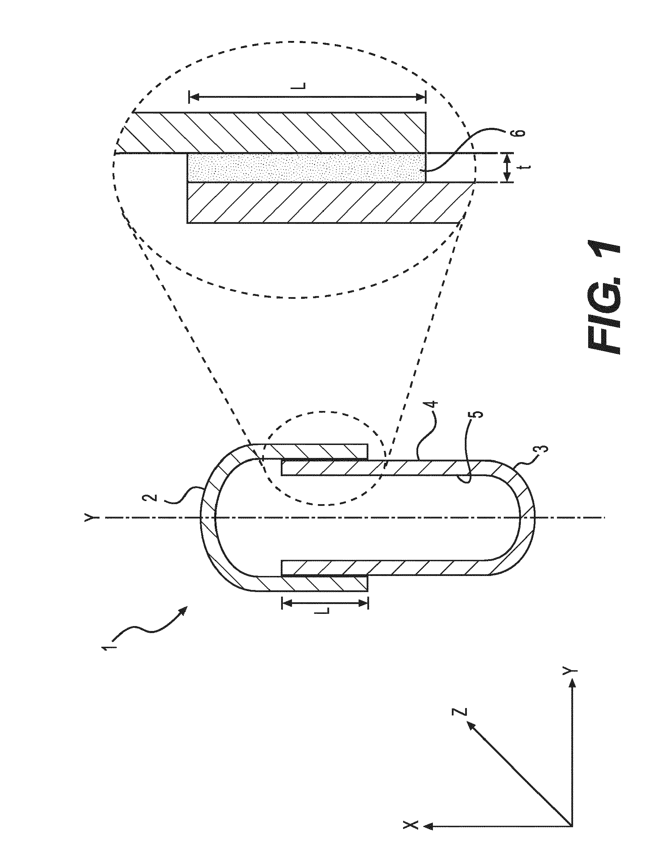

Fig. 1 is an illustration of a cross-sectional view of a dosage form article according to one aspect of the disclosure.

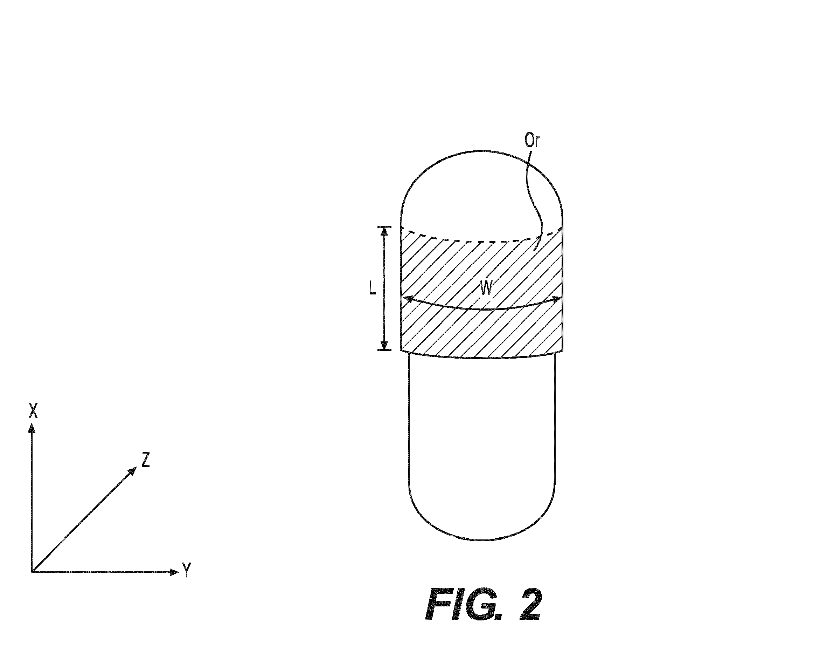

Fig. 2 is a perspective representation of a dosage form article according to one aspect of the disclosure.

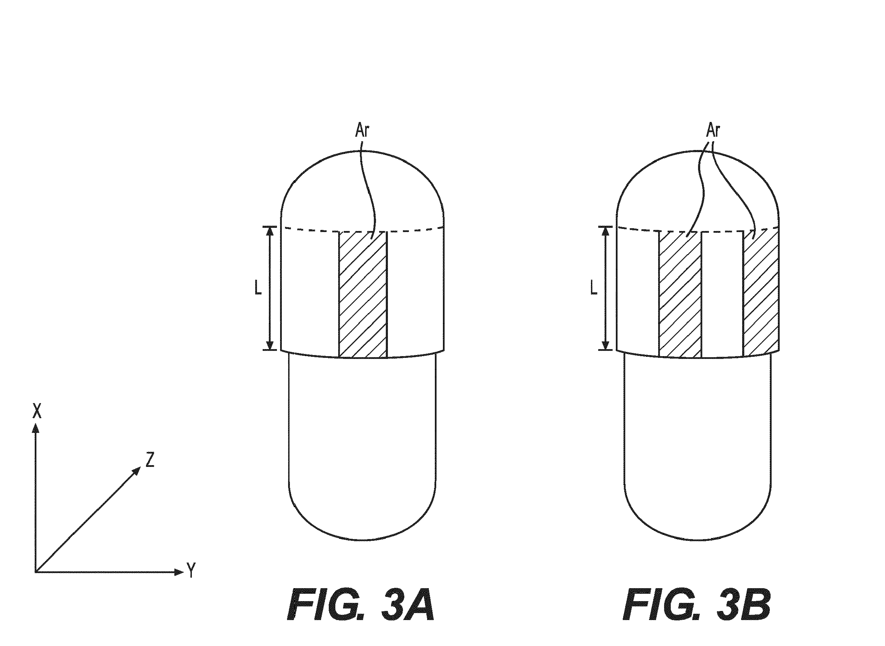

Fig. 3 (A and B) is a perspective representation of a dosage form article according to one aspect of the disclosure.

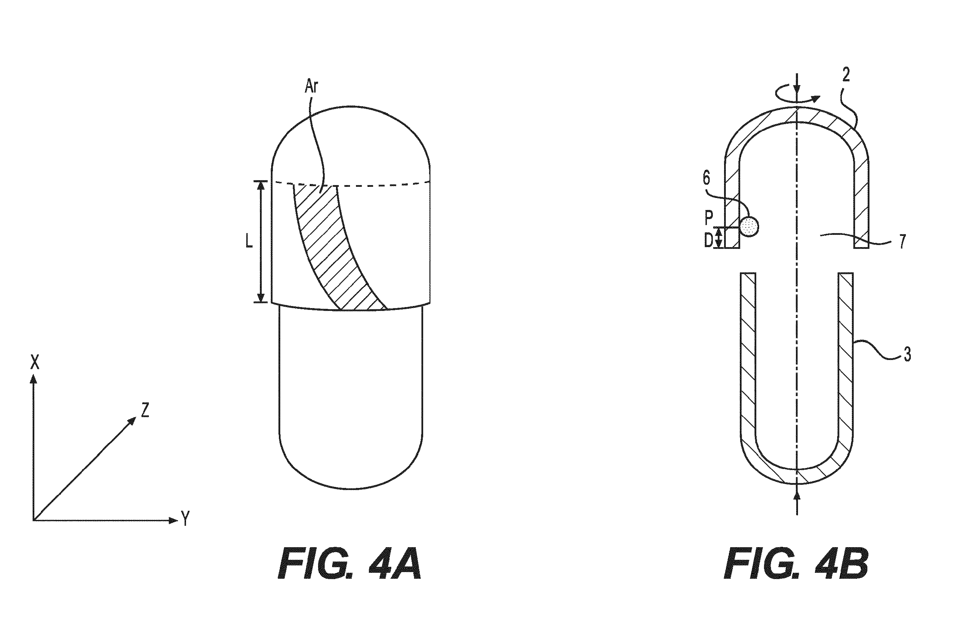

Fig. 4A is a perspective representation of a dosage form article according to one aspect of the disclosure.
Fig. 4B is a cross-sectional view of a dosage form article illustrating the process steps to generate the dosage form of Fig. 4A.

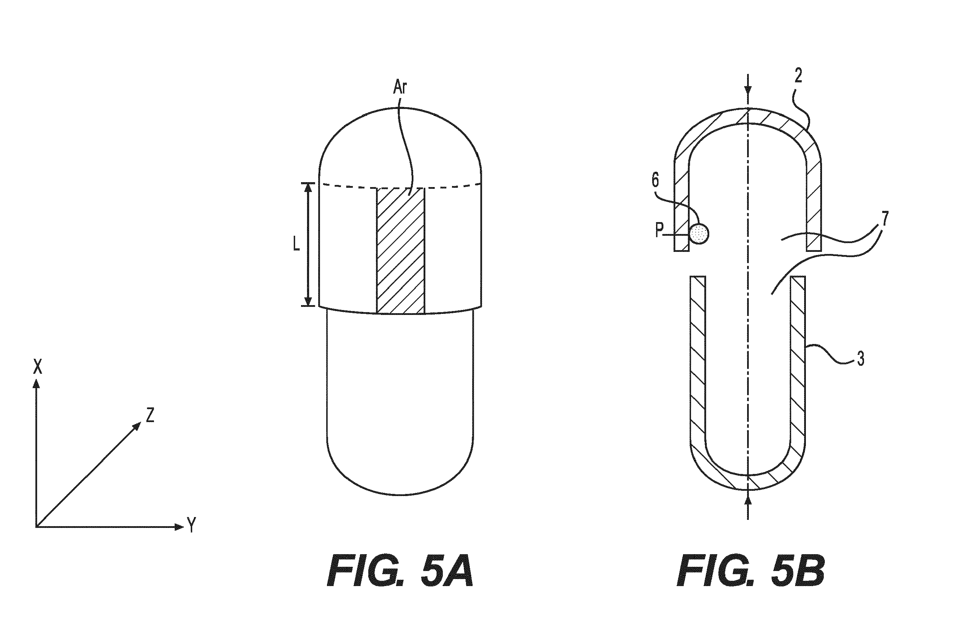

Fig. 5A is a perspective representation of a dosage form article according to one aspect of the disclosure.
Fig. 5B is a cross-sectional view of a dosage form article illustrating the process steps to generate the dosage form of Fig. 5A.

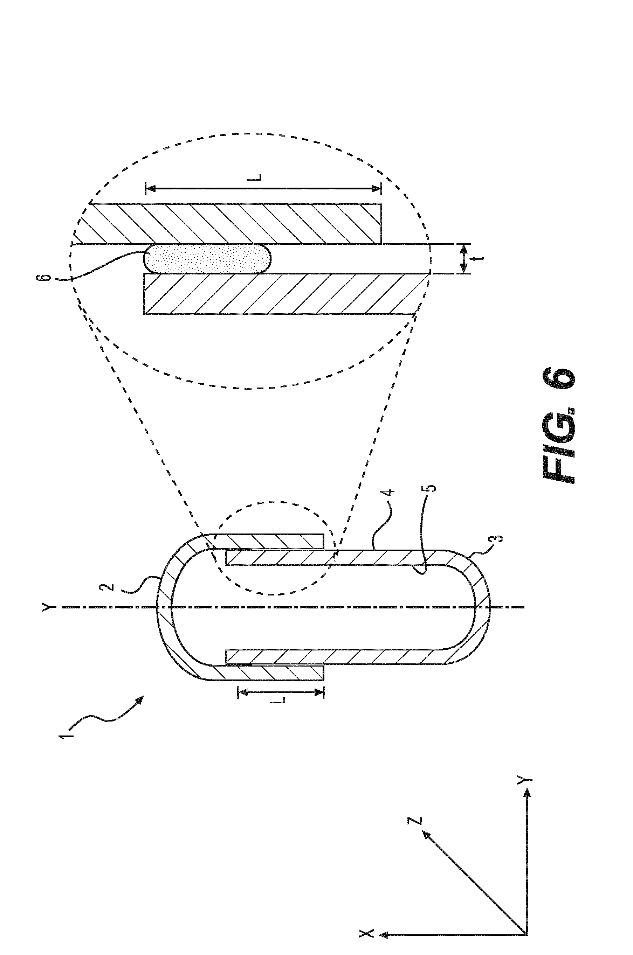

Fig. 6 is an illustration of a cross-sectional view of a dosage form article according to one aspect of the disclosure.

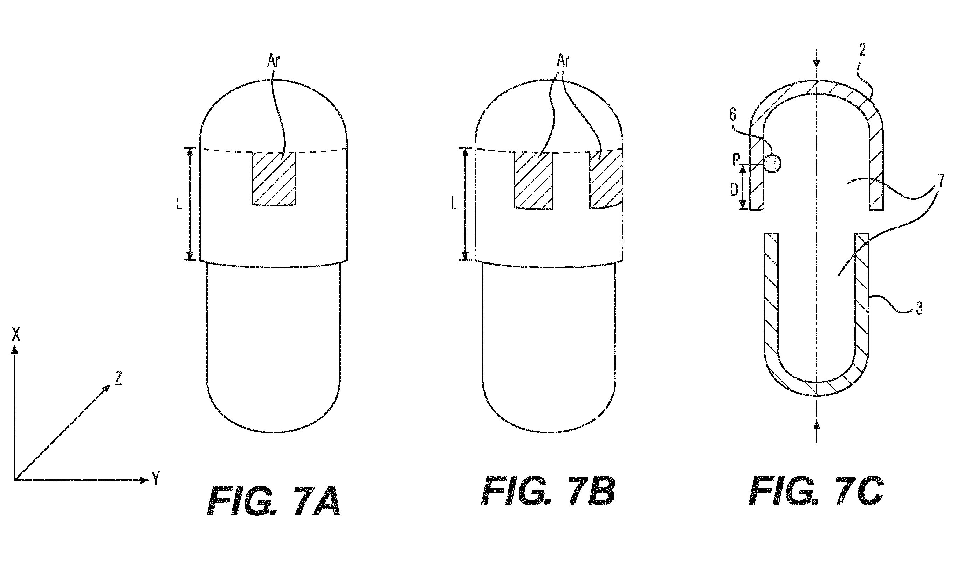

Fig. 7A is a perspective representation of a dosage form article according to one aspect of the disclosure.
Fig. 7B is a perspective representation of a dosage form article according to one aspect of the disclosure.
Fig. 7C is a cross-sectional view of a dosage form article illustrating the process steps to generate the dosage form of Fig. 7A and 7B.

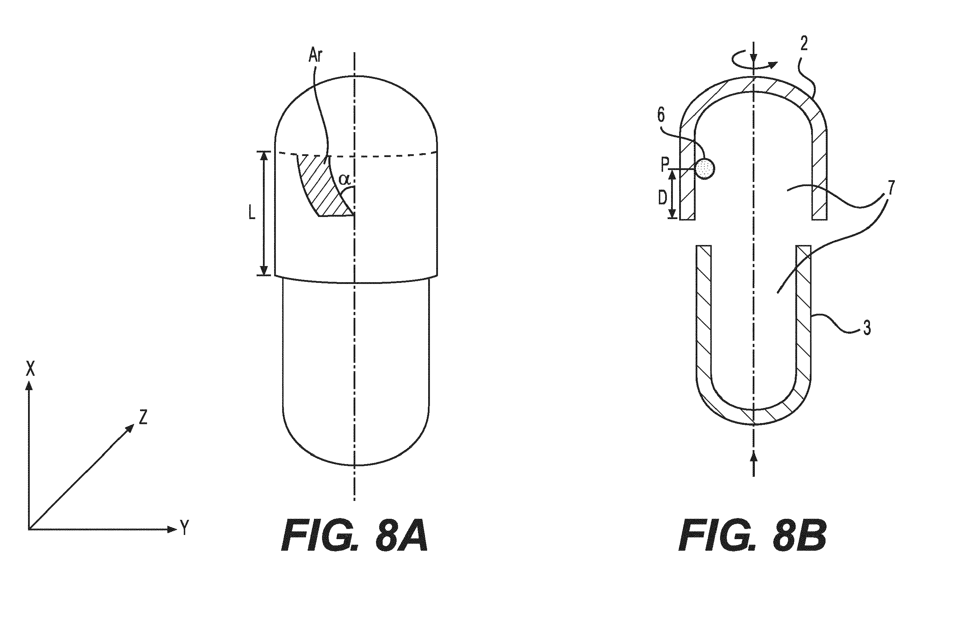

Fig. 8A is a perspective representation of a dosage form article according to one aspect of the disclosure.
Fig. 8B is a cross-sectional view of a dosage form article illustrating the process steps to generate the dosage form of Fig. 8A.

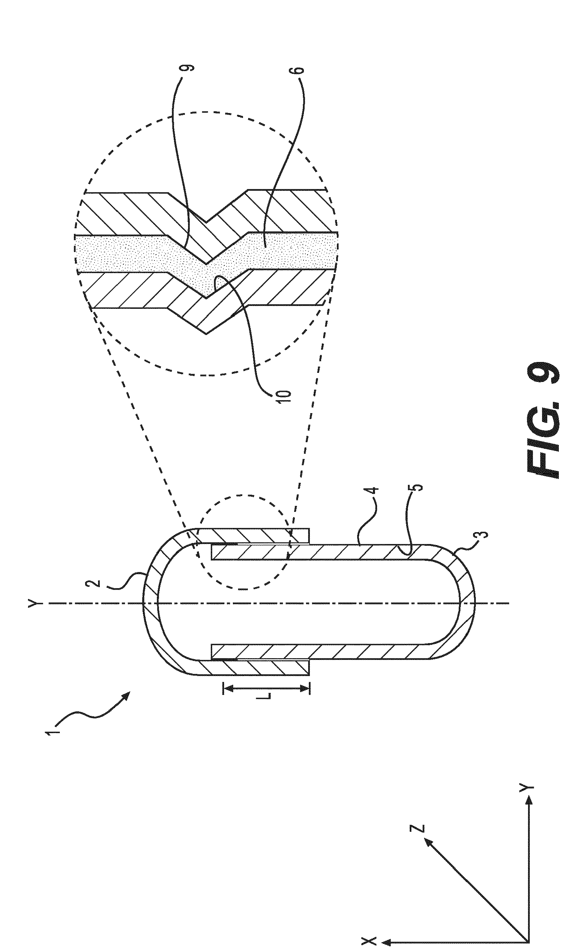

Fig. 9 is an illustration of a cross-sectional view of a dosage form article according to one aspect of the disclosure.

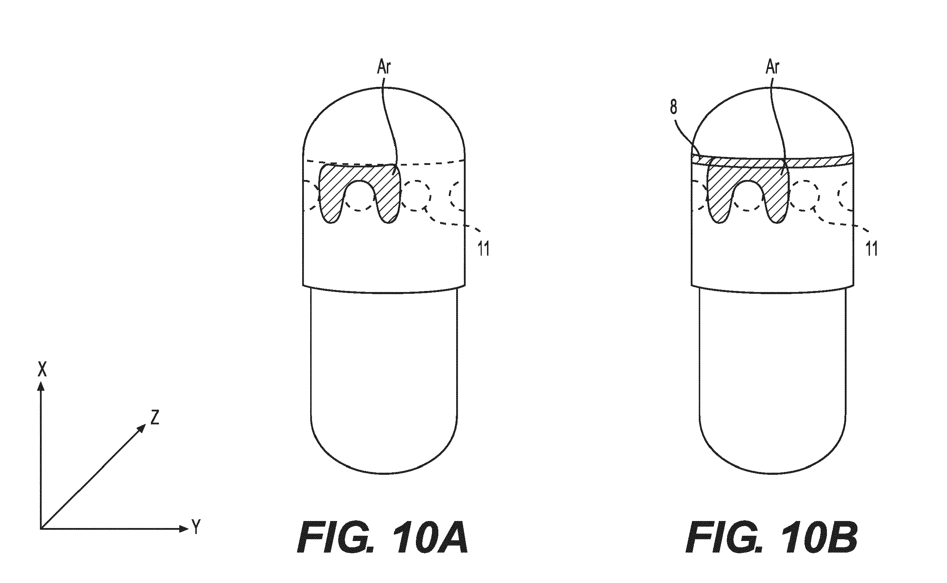

Fig. 10 (A and B) is a perspective representation of a dosage form article according to one aspect of the disclosure.
Fig. 11 is an image illustrating the set-up used to measure the pull-apart forces herein described.
Fig. 12 is a graph illustrating the pull-apart force variation with %wt of adhesion enhancing agent in the adhesive substance.
Fig. 13 is a graph illustrating the deformation observed with %wt of water in the adhesive substance.
Fig. 14 is an image illustrating the set-up used to measure torsion herein described.

### DETAILED DESCRIPTION

By the term "a" and/or "an" when describing a particular element, it is intended "at least one" of that particular element.

By the term "medicament", it is intended a "drug" or the like comprising one or more compounds providing one or more curative benefits to a subject, the terms "medicament" and "drug" may be used interchangeably herein.

By the term "hard shell" or "hard capsule shell", it is intended a shell that is deformable, but which returns to its un-deformed shape upon the removal of a deforming force. Typically such shells comprise less than 25%, preferably less than 20%, more preferably from 0% to 14%, even more preferably from greater than 0% to less than 14%, water by weight.

By the term "free of organic solvent", it is intended that the level of organic solvent does not exceed 5%, preferably 2%, preferably 1.5%, more preferably 1%, even more preferably 0.5%, even more preferably 0.2%, most preferably about 0%, by weight of the item referred to (e.g. by weight of the composition).

By the term "tamperproof" or "tamperproofness", it is intended that the opening force is greater or equal to the fracture strength (also commonly referred to as breaking strength) of the dosage form. Generally, the fracture strength of a dosage form may be determined by graphical analysis of a stress-strain curve following a tensile test. Typically, such opening force is greater than 20N, preferably greater than 40N, more preferably from 60N to 100N, according to the method described herein. Additionally or alternatively, such opening force may also convert to a torsion force that is greater than 40Nmm, preferably greater than 80Nmm, more preferably from 120Nmm to 450Nmm.

Various embodiments will now be described to provide an overall understanding of the principles of the structure, function, manufacture, and use of dosage form articles and methods disclosed herein. One or more examples of these embodiments are illustrated in the accompanying figures. Those of ordinary skill in the art will immediately understand that features described or illustrated in connection with one example embodiment can be combined with the features of other example embodiments without generalization from the present disclosure.

### DOSAGE FORM ARTICLES

In an embodiment, a tamperproof dosage form **1** for oral administration is contemplated. The dosage form **1** comprising: a cap **2** and a body **3** each comprising an outer surface **4** and an inner surface **5,** the cap **2** and body **3** being arranged to telescopically engage with each other such that an overlap region **Or** is formed between a portion of the outer surface **4** of the body **3** and a portion of the inner surface **5** of the cap **2,** said overlap region **Or** comprising an overlap length **L** extending parallel to a centerline **Y** of said dosage form **1,** wherein an adhesive substance **6** is comprised throughout substantially the entire overlap length **L** bonding said cap **2** to said body **3.** The adhesive **6** is generally arranged to directly bond at least a portion of the outer surface **4** of the body **3** with least a portion of the respective inner surface **5** of the cap **2,** by typically being located between the outer surface **4** of the body **3** and the inner surface **5** of the cap **2.**

The overlap region **Or** may have an overlap width **W** that extends along a circumference of said dosage form **1** and the adhesive substance **6** may be comprised in an adhesive region **Ar,** said adhesive region **Ar** extending throughout substantially the entire overlap length **L** and at least a portion of the overlap width **W,** preferably less than the total overlap width **W,** the total overlap width **W** typically being equal to the total circumference of the dosage form taken along a plane perpendicular to the centerline **Y.**

The adhesive region **Ar** may comprise an adhesive area that is at least 5%, preferably at least 10%, more preferably at least 15%, even more preferably at least 20%, of an overlap area of the overlap region **Or.** Respective areas referred to herein above are preferably surface areas of the respective regions taken from a plane parallel to the centerline **Y.**

The adhesive region **Ar** may comprise an adhesive thickness **t** extending along an axis perpendicular to the centerline **Y,** said thickness **t** being is less than 1%, preferably from greater than 0% to less than 0.5%, of the overlap length **L.** The adhesive thickness **t** may be arranged to, preferably completely, fill a gap formed between the outer surface **4** of the body and the inner surface **5** of the cap **2.** The thickness **t** may extend substantially perpendicular to the adhesive area. An adhesive volume may be determined by multiplying the adhesive area by the thickness **t.**

The tamperproof dosage form **1** may comprise a plurality of adhesive regions **Ar** discontinuously or continuously, preferably discontinuously, extending along the overlap width **W.** The plurality of adhesive regions **Ar** are, preferably equally, spaced apart from one another along the overlap width **W** such that the distance between each of a neighboring adhesive region **Ar** along the overlap width **W** is less than or equal to 50%, preferably less than or equal to 25%, more preferably from greater than 20% to 50%, of the total overlap width **W.**

The adhesive substance **6** may be aqueous (i.e. comprises water, or consists of water) and/or free of organic solvents and may comprise one or more adhesive enhancing agents. The adhesive enhancing agent may be selected from the group consisting of one or more polysaccharides, generally celluloses typically selected from the group consisting of hydroxypropylcellulose, hydroxypropylmethylcellulose, sodiumcarboxymethylcellulose, and mixtures thereof, chitosan, sugars such as sucrose, fructose, lactose, maltose, cellobiose, glucose, galactose, mannose, arabinose, sorbitol, and mixtures thereof, potato and/or corn starch, aspartame, glycosides such as steviol, synthetic homopolymers of N-vinyl-2pyrrolidone; gelatin (bovine and/or porc); monofunctional organic acid such as fatty acids, acetic acid, benzoic acid, propanoic acid, and mixtures thereof; polyfuntional organic acids such as citric acid, glycolic acid, lactic acid, mailic acid, tartaric acid, mandelic acid, fumaric acid, phosphoric acid and mixtures thereof; and mixtures thereof. Preferably the adhesive enhancing agent is selected from the group consisting of sucrose, fructose, lactose, maltose, cellobiose, glucose, galactose, mannose, arabinose, sorbitol, and mixtures thereof. Further details about suitable adhesives are provided in the following sections.

In an embodiment, the tamperproof dosage form **1** for oral administration may comprise: a cap **2** and a body **3** each comprising an outer surface **4** and an inner surface **5,** the cap **2** and body **3** being arranged to telescopically engage with each other such that an overlap region **Or** is formed between a portion of the outer surface **4** of the body **3** and a portion of the inner surface **5** of the cap **2,** said overlap region **Or** comprising an overlap length **L** extending parallel to a centerline **Y** of said dosage form **1,** wherein an adhesive substance **6** is comprised throughout at least a portion of the overlap length **L** bonding said cap **2** to said body **3,** wherein said adhesive substance is free of organic solvents and may comprise one or more adhesion enhancing agents. The adhesive **6** is generally arranged to directly bond at least a portion of the outer surface **4** of the body **3** with least a portion of the respective inner surface **5** of the cap **2,** by typically being located between the outer surface **4** of the body **3** and the inner surface **5** of the cap **2.** The overlap region **Or** may have an overlap width **W** that extends along a circumference of said dosage form **1** and the adhesive substance **6** is comprised in an adhesive region **Ar,** said adhesive region **Ar** extending throughout at least a portion of the, preferably substantially the entire, overlap length **L** and at least a portion of the overlap width **W,** preferably less than the total overlap width **W.** The adhesive substance **6** may be comprised throughout at least 50%, preferably at least 60%, more preferably at least 70%, even more preferably at least 80%, most preferably about 100% of the overlap length **L.**

In an embodiment, the adhesive substance extends in the overlap region **Or** along only a portion of the overlap length **L** (i.e. not the entire length **L**) and extends from a position proximal to the opening **7** of the body **3** (and distal from the opening **7** of the cap **2**) towards the opening **7** of the cap **2.** In this embodiment, the dosage form may further comprise at least one mechanical locking feature **8** that is preferably arranged proximal to a closed end of the cap **2** and distal to an open end of the cap **2,** preferably in the form of one or more protrusions **9** and one or more recesses **10** (commonly in the form of locking rings for locking engagement), wherein generally the adhesive substance **6** is disposed in said mechanical locking feature **8,** preferably between said protrusions **9** and said recesses **10,** typically a portion of or the entire surface of the locking feature **8.** Without wishing to be bound by theory it is believed that by disposing an adhesive substance to only a part of the overlap length in a position as described above allows for the adhesive substance to be well distributed in the overlap gap proximal to the locking feature thanks to capillary action in the locking feature surfaces, thus resulting in similar tamperproof properties as when the adhesive is present along substantially the entire overlap length.

In an embodiment, the cap may comprise a plurality of discontinuous protrusions **11,** preferably circular or substantially elliptical in shape, extending along a circumference thereof. In this embodiment, the adhesive region **Ar** may extend through and/or between at least two consecutive said discontinuous protrusions **11.** Such arrangement has the advantage of providing further resistance to torsion forces as well as even distribution of the adhesive substance by localized capillary action.

In any of the embodiments herein, the adhesive region **Ar** may extend along length **L** following a substantially linear path parallel to the centerline **Y.** This has the advantage of good resistance to torsion during attempt tampering by rotation of the cap relative to the body. Alternatively, the adhesive region **Ar** may extend along length **L** following a substantially non-linear, preferably skewed, path to the centerline **Y.** Preferably the adhesive region **Ar** is at an angle **α** to the centerline **Y.** The angle **α** may be from greater than 0°to less than 90°, preferably from greater t han 5°to less than 80°, more preferably from greater than 10° to less than 70°, even more preferably from greater than 15° to less than 60°. The advantage of this configuration is to further provide good resistance to tension forces during attempt tampering by pull-apart of the cap relative to the body in a direction substantially parallel to the centerline **Y,** as well as good resistance to torsion during attempt tampering by rotation of the cap relative to the body.

In an embodiment (not shown), the dosage form article may further comprise a fluid stop joint located proximal to the open end of the cap and distal to the closed end of the cap. The fluid stop joint may be arranged as described in paragraphs 0038 and 0039 of US2007/0184077A1. In this embodiment the dosage form may be further sealed with a second adhesive substance that may comprise a mixture of water and at least one organic solvent and applied as described in paragraph 0037 of US2007/0184077A1. Unlike the adhesive substance described herein above, the second adhesive substance may indeed comprise an organic solvent since the fluid stop joint ensures that the second adhesive substance remains located proximal to the open end of the cap and prevents alcohol vapors from penetrating further in the overlap region along the overlap length, which would otherwise result in excessive brittleness and deformation. An advantage of this embodiment is to combine the tamperproofness benefits of the above embodiments together with the added sealing benefits of the present embodiment (particularly when liquids are to be stored in the dosage form) without compromising on structural stability (i.e. no deformation or failure due to liquid sealing). In this arrangement one or more adhesive regions are formed extending a portion of the overlap length preferably up to the fluid stop joint, and a sealing region is formed along the entire circumference of the dosage form along a portion of the overlap length typically from the fluid stop joint up to a rim of the open end of the cap.

In any of the embodiments herein, the dosage form article may comprise indicia to, typically visually, or otherwise, indicate whether the dosage from has been tampered with. The indicia may be in the form of adhesive substances described herein being arranged such that it may be seen through the cap and/or body (for example by inclusion of one or more tribochromatic compounds in the adhesive substances herein as explained in more detail in the section that follows). Alternatively, or in addition, the cap and/or body may be translucent such that the indicia may be seen through the cap and/or body. The tribochromatic compounds for use herein above may be capable of changing color, preferably irreversibly, upon the application of shear. In these arrangements, the dosage form may be inspected before ingesting and provide a warning to the patient if the dosage form has been tampered.

In an embodiment the dosage form articles herein are multi-piece capsules comprising a plurality of capsule shells (selected from cap(s) and/or body(s)). The capsule shells may each comprise locking features to mechanically lock with one or more other capsule shells. Said features may comprise a combination of protrusions and recesses of complementary shape such that when interposed lock the capsule shells together. Such locking features may provide additional tamperproof resistance to the adhesives described herein.

The dosage form articles herein, preferably the shells thereof, may be made of, or consist of, an ingestible material comprising materials selected from the group consisting of gelatin, one or more polysaccharides, preferably pullulan; nonionic hydrogels, preferably cellulose such as hydroxypropyl methylcellulose (HPMC); and mixtures thereof. Most preferred materials being gelatin and/or hydroxypropyl methylcellulose (HPMC). Dosage form articles herein may be non-injection molded, and preferably made via a dip molding process. The latter ensures high production speeds and cost effectiveness. Other materials may also be used, as will be recognized by one skilled in the art, including cellulose ethers, such as starches (e.g. waxy maize starch, tapioca dextrin, and derivatives thereof), carrageenan, and polymers or copolymers of (meth)acrylic acids and derivatives thereof.

Typically, the cap and body parts may be substantially tubular in shape and each comprise a single opening. The cap and/or body parts described herein may be hard capsule shells.

In an embodiment, the dosage forms herein are not banded. An advantage of this arrangement is to retain the tamperproof benefits whilst maintaining good visual acceptance by subjects of the dosage form.

Dosage form articles described herein are particularly useful in dry-powder-inhalation (DPI) applications wherein the dosage form is inserted into an inhalation device that typically retains and pierces the dosage form to allow its content to be inhaled. Indeed, it has been found that dosage forms of the state of the art may accidentally open during handling prior to insertion into the inhalation device or during the insertion process. This issue has been found greatly problematic when a pre-defined dose of the contents must be delivered to the subject. The use of dosage forms described herein in said applications ensure that the dosage form remains closed and secured at all times prior to and during insertion and piercing in an inhalation device.

### ADHESIVE

Adhesive compositions for use herein are those suitable for bonding one or more segments selected from a cap and body of a tamperproof dosage form for oral administration as described herein. The adhesive composition is typically an aqueous composition free of organic solvents and may comprise or consist essentially of, one or more adhesive enhancing agents and water.

The adhesive composition may consist essentially of one or more adhesive enhancing agents, water and an indicia imparting compound. Such is particularly desirable when the dosage form comprises indicia for indicating whether the dosage form has been tampered with.

Water is preferably comprised at a level of from greater than 50%, typically greater than 70% or even greater than 80%, preferably from 50% to 100%, preferably from 70% to 95%, more preferably from 80% to 94%, even more preferably from 88% to 93%, by weight of said composition.

The adhesive enhancing agent may be selected from the group consisting of one or more polysaccharides, generally celluloses typically selected from the group consisting of hydroxypropylcellulose, hydroxypropylmethylcellulose, sodiumcarboxymethylcellulose, and mixtures thereof, chitosan, sugars such as sucrose, fructose, lactose, maltose, cellobiose, glucose, galactose, mannose, arabinose, sorbitol, and mixtures thereof, potato and/or corn starch, aspartame, glycosides such as steviol, synthetic homopolymers of N-vinyl-2pyrrolidone; gelatin (bovine and/or porc); monofunctional organic acid such as fatty acids, acetic acid, benzoic acid, propanoic acid, and mixtures thereof; polyfuntional organic acids such as citric acid, glycolic acid, lactic acid, mailic acid, tartaric acid, mandelic acid, fumaric acid, phosphoric acid and mixtures thereof; and mixtures thereof. Preferably the adhesive enhancing agent is selected from the group consisting of sucrose, fructose, lactose, maltose, cellobiose, glucose, galactose, mannose, arabinose, sorbitol, and mixtures thereof. Further details about suitable adhesives are provided in the following sections.

In an embodiment, the adhesive enhancing agent consists essentially of one or more polysaccharides, preferably celluloses and/or sugards, and one or more monofunctional or polyfuntional organic acids.

Typically, the adhesive enhancing agent is comprised at a level of from 0% to 50%, preferably from greater than 0% to 50%, preferably from 2% to 40%, more preferably from 4% to 30%, more preferably from 5% to 20%, even more preferably from 7% to 12%, by weight of the composition.

In an embodiment, the ratio of the one or more adhesive enhancing agents to water is from 0.05:1.00 to 0.45:1.00, preferably fom 0.05:1.00 to 0.25:1.00, even more preferably from 0.08:1 to 0.13:1.00. Surprisingly, such ratios ensure good adhesion not only when gelatin is used as material for the dosage form shells (cap and body) but also when other polymer based materials are used, such as polysaccharides and celluloses as described above.

The adhesive composition is preferably a low viscosity and/or non-pasty adhesive composition, preferably having a viscosity of from 0.5 mPa•s to 1300 mPa•s, preferably from 0.7 mPa•s to 1200 mPa•s, more preferably from 0.9 mPa•s to 1100 mPa•s or even from 1 mPa•s to 1000 mPa•s. The viscosity generally being measured at room temperature (about 22.5°C) with a Brookfield type LVDV II+, spindle 21, at 100 RPM.

The indicia imparting compound may be selected from one or more optional tribochromatic (i.e. tribochromic) compounds, coloring agent(s), and mixtures thereof.

Tribochromic compounds are colored compounds that have the property of changing color when subjected to mechanical stress, such as shear. When this compound is impregnated or applied onto a support, the color of the support will undergo a color change at the time of the mechanical stress/shear. Typical forces to be applied in order to obtain the desired tribochromic effect range from 0.01 N to 50 N, for instance from 0.1 N to 20 N, such as from 1 N to 20N. This color change is irreversible. Once the conformation of the molecule has been modified, the latter cannot return to its initial conformation.

The color shade obtained depends on the force and the time of the mechanical stress exerted on the material. The longer and/or greater the mechanical stress, the greater the amount of tribochromic compounds whose conformation will have changed and the greater the modification of the color or the shade.

Suitable tribochromic compounds for use herein are described in paragraph 0028 to 0035 of US2006/0286049A1.

The one or more tribochromic compounds may be used alone or in combination with other colorants or pigments to provide different visual effects.

Suitable coloring agents for use herein include pharmaceutically acceptable coloring agents, food acceptable colorants, or mixtures thereof. Examples of such colorants include, but are not limited to, azo-, quinophthalone-, triphenylmethane-, xanthene- or indigoid dyes; iron oxides or hydroxides; titanium dioxide; natural dyes; and mixtures thereof. Additional examples include patent blue V, acid brilliant green BS, red 2G, azorubine, ponceau 4R, amaranth, D+C red 33, D+C red 22, D+C red 26, D+C red 28, D+C yellow 10, yellow 2 G, FD+C yellow 5, FD+C yellow 6, FD+C red 3, FD+C red 40, FD+C blue 1, FD+C blue 2, FD+C green 3, brilliant black BN, carbon black, iron oxide black, iron oxide red, iron oxide yellow, titanium dioxide, riboflavin, carotenes, anthocyanines, turmeric, cochineal extract, chlorophyllin, canthaxanthin, caramel, betanin and CANDURIN® pearlescent pigments. CANDURIN ® is manufactured and marketed by Merck KGaA, Darmstadt, Germany and consists of titanium dioxide and/or iron oxide (approved food and pharmaceutical colorants in many countries) and potassium aluminum silicate as a color carrier.

The indicia imparting compound may be comprised at a level of from 0% to 5%, preferably from greater than 0% to 3%, more preferably from greater than 0% to 2%, by weight of the adhesive composition.

Preferably, the adhesives herein are used for bonding a cap and a body of a dosage form to provide tamperproofness of the same.

In cases where the pressure differential caused during closing of the dosage forms disrupts the location and spreading of the adhesive, the adhesive substance may be adapted to improve/increase surface tension and/or viscosity accordingly. In an embodiment, the adhesive substance further comprises a surface tension modifier selected from one or more organic solvents (such as ethanol), one or more surfactants, and mixtures thereof.

### DRUG / MEDICAMENT

Dosage form articles described herein may comprise one or more drugs. Drugs suitable for use in the dosage forms described herein may take any form and be for any treatment of a human or animal subject. This includes not only pharmaceutical compounds but also dietary supplements such as vitamins, minerals and the like.

The drug may be in a state selected from solid or liquid, at room temperature and atmospheric pressure, and comprises one or more active compounds. The physical state of said drug is typically wholly dependent on the needs for a given application. When the drug is in solid state the drug may be powder-like or caplet-like (i.e. tablet-like). In an embodiment, the drug is in the form of a caplet or tablet typically having a first and second end. The caplet or tablet may be further coated, alternatively or additionally, at least one, preferably both, first and second ends of the caplet or tablet may be coated with capsule shells. The capsule shells may be gelatin comprising or hydroxypropyl methylcellulose (HPMC) comprising shells. By "capsule shells" it is herein intended at least portions of body or cap parts of hard capsule shells cut to size such to fit and insert over the outer surface of the caplet or tablet to provide a tight fit when joined.

Suitable compounds for delivery according to the disclosure include, but are not limited to, powder, liquid, and/or pellet forms of the following:
a) pharmaceuticals (also called pharmaceutical actives) such as betamethasone, thioctic acid, sotalol, salbutamol, norfenefrine, silymahn, dihydroergotamine, buflomedil, etofibrate, indomethacin, oxazepam, acetyldigitoxins, piroxicam, halopehdol, isosorbide mononitrate, amithptyline, diclofenac, nifedipine, verapamil, pyritinol, nitrendipine, doxycycline, bromhexine, methylprednisolone, clonidine, fenofibrate, allopurinol, pirenzepine, levothyroxine, tamoxifen, metildigoxin, o-(B-hydroxyethyl)-rutoside, propicillin, aciclovirmononitrate, paracetamolol, naftidrofuryl, pentoxifylline, propafenone, acebutolol, 1-thyroxin, tramadol, bromocriptine, loperamide, ketofinen, fenoterol, ca-dobesilate, propranolol, minocycline, nicergoline, ambroxol, metoprolol, B-sitosterin, enalaprilhydrogenmaleate, bezafibrate, isosorbide dinitrate, gallopamil, xantinolnicotinate, digitoxin, flunitrazepam, bencyclane, depanthenol, pindolol, lorazepam, diltiazem, piracetam, phenoxymethylpenicillin, furosemide, bromazepam, flunarizine, erythromycin, metoclopramide, acemetacin, ranitidine, biperiden, metamizol, doxepin, dipotassiumchlorazepat, tetrazepam, estramustinephosphate, terbutaline, captopril, maprotiline, prazosin, atenolol, glibenclamid, cefaclor, etilefrin, cimetidine, theophylline, hydromorphone, ibuprofen, primidone, clobazam, oxaceprol, medroxyprogesterone, flecainide, Mgpyhdoxal-5-phosphateglutaminate, hymechromone, etofyllineclofibrate, vincamine, cinnarizine, diazepam, ketoprofen, flupentixol, molsidomine, glibornuhde, dimethindene, melperone, soquinolol, dihydrocodeine, clomethiazole, clemastine, glisoxepid, kallidinogenase, oxyfedhne, baclofen, carboxymethylcystsin, thioredoxin, betahistine, 1-tryptophan, myrtol, bromelain, prenylamine, salazosulfapyridine, astemizole, sulpiride, benzerazid, dibenzepin, acetylsalicylic acid, miconazole, nystatin, ketoconazole, sodium picosulfate, colestyramate, gemfibrozil, rifampin, fluocortolone, mexiletine, amoxicillin, terfenadine, mucopolysaccharidpolysulfuric acid, triazolam, mianserin, tiaprofensaure, ameziniummethylsulfate, mefloquine, probucol, quinidine, carbamazepine, Mg-1-aspartate, penbutolol, piretanide, amitriptyline, caproteron, sodium valproinate, mebeverine, bisacodyl, 5-amino-salicyclic acid, dihydralazine, magaldrate, phenprocoumon, amantadine, naproxen, carteolol, famotidine, methyldopa, auranofine, estriol, nadolol, levomepromazine, doxorubicin, medofenoxat, azathioprine, flutamide, norfloxacin, fendiline, prajmaliumbitartrate, aescin acromycin, anipamil, benzocaine, [beta]- carotene, cloramphenicol, chlorodiazepoxid, chlormadinoneacetate, chlorothiazide, cin- narizine, clonazepam, codeine, dexamethasone, dicumarol, digoxin, drotaverine, grami- cidine, griseofulvin, hexobarbital hydrochlorothiazide, hydrocortisone, hydroflumethiazide, ketoprofen, lonetil, medazepam, mefruside, methandrostenolone, sulfaperine, nalidixic acid, nitrazepam, nitrofurantoin, estradiol, papaverine, phenacetin, phenobarbi- tal, phenylbutazone, phenytoin, prednisone, reserpine, spironolactine, streptomycin, sul- famethizole, sulfamethazine, sulfamethoxoazole, sulfamethoxydiazinon, sulfathiazole, sulfisoxazole, testosterone, tolazamide, tolbutamide, trimethoprim, tyrothricin, antacids, reflux suppressants, antiflatulents, antidopaminergics, proton pump inhibitors, H2- receptor antagonists, cytoprotectants, prostaglandin analogues, laxatives, antispasmodics, antidiarrhoeals, bile acid sequestrants, opioids, beta-receptor blockers, calcium channel blockers, diuretics, cardiac glycosides, antiarrhythmics, nitrates, antianginals, vasoconstrictors, vasodilators, ACE inhibitors, angiotensin receptor blockers, alpha blockers, anticoagulants, heparin, antiplatelet drugs, fibrinolytic, anti-hemophilic factor, haemostatic drugs, hypolipidaemic agents, statins, hypnotics, anaesthetics, antipsychotics, antidepressants (including tricyclic antidepressants, monoamine oxidase inhibitors, lithium salts, selective serotonin reuptake inhibitors), anti-emetics, anticonvulsants, an- tiepileptics, anxiolytics, barbiturates, movement disorder drugs, stimulants (including amphetamines), benzodiazepine, cyclopyrrolone, dopamine antagonists, antihistamines, cholinergics, anticholinergics, emetics, cannabinoids, 5-HT antagonists, analgesics, muscle relaxants, antibiotics, sulfa drugs, aminoglycosides, fluoroquinolones, bronchodilators, NSAIDs, anti-allergy drugs, antitussives, mucolytics, decongestants, corticosteroids, beta-receptor antagonists, anticholinergics, steroids, androgens, antian- drogens, gonadotropin, corticosteroids, growth hormones, insulin, antidiabetic drugs (including sulfonylurea, biguanide/metformin, and thiazolidinedione), thyroid hormones, antithyroid drugs, calcitonin, diphosponate, vasopressin analogs, contraceptives, follicle stimulating hormone, luteinising hormone, gonadotropin release inhibitor, progestogen, dopamine agonists, oestrogen, prostaglandin, gonadorelin, clomiphene, tamoxifen, di- ethylsti I bestrol , antimalarials, anthelmintics, amoebicides, antivirals, antiprotozoals, vaccines, immunoglobulin, immunosuppressants, interferon, monoclonal antibodies, and mixtures thereof;
b) vitamins, e.g., fat-soluble vitamins such as vitamins A, D, E, and K, and water soluble vitamins such as vitamin C, biotin, folate, niacin, pantothenic acid, riboflavin, thiamin, vitamin B6, vitamin B12, and mixtures thereof;
c) minerals, such as calcium, chromium, copper, fluoride, iodine, iron, magnesium, manganese, molybdenum, phosphorus, potassium, selenium, sodium (including sodium chloride), zinc, and mixtures thereof;
d) dietary supplements such as herbs or other botanicals, amino acids, and substances such as enzymes, organ tissues, glandulars, and metabolites, as well as concentrates, metabolites, constituents, extracts of dietary ingredients, and mixtures thereof;
e) homoeopathic ingredients such as those listed in the Homeopathic Pharmacopoeia of the United States Revision Service (HPRS), and mixtures thereof. It must be recognized, of course, that the HPRS is periodically updated and that the present invention includes homeopathic ingredients that may be added to the HPRS; and mixtures in any combination of the foregoing.

### PROCESS OF MAKING

The process of making a tamperproof dosage form **1** herein may comprise the steps of: providing a cap **2** and a body **3** each comprising an outer surface **4** and an inner surface **5;** applying an adhesive substance **6,** preferably consisting of adhesive compositions described herein, on the inner surface **5** of the cap **2** or the outer surface **4** of the body **3** or both; and telescopically inserting the cap **2** over the body **3** such that the adhesive substance is distributed throughout substantially an entire overlap length L once the cap **2** and body **3** reach a fully inserted position.

The adhesive substance may be applied proximal to an open end **7** of the cap **2** or body **3** or both prior to telescopically inserting the cap **2** over the body **3,** preferably wherein the adhesive substance is applied in the form of a droplet, preferably having a diameter of from typically having a diameter of from 0.1 mm to 2 mm, preferably from 0.1 mm to 1 mm, preferably from 0.2mm to 0.8mm, more preferably from 0.3mm to 0.7mm, more preferably from 0.4mm to 0.7mm.

In an embodiment, the process of making a tamperproof dosage form **1** herein may comprise the steps of: providing a cap **2** and a body **3** each comprising an outer surface **4** and an inner surface **5;** applying an adhesive substance **6,** preferably consisting of the adhesive compositions herein, on the inner surface of the cap **2** or the outer surface of the body **3** or both; and telescopically inserting the cap **2** over the body **3** such that the adhesive substance is distributed throughout at least a portion of overlap length **L** once the cap **2** and body **3** reach a fully inserted position.

The adhesive substance **6** may be applied at a position **P,** wherein said position **P** is at a distance D from an open end **7** of the cap **2** or body **3** prior to telescopically inserting the cap **2** over the body **3,** and said distance **D** is from 0% to less than 50%, preferably from 1% to 35%, more preferably from 2% to less than 30%, of the overlap length **L,** preferably wherein the adhesive substance is applied in the form of a droplet, typically having a diameter of from 0.1 mm to 2 mm, preferably from 0.1 mm to 1 mm, preferably from 0.2mm to 0.8mm, more preferably from 0.3mm to 0.7mm, more preferably from 0.4mm to 0.7mm.

In an embodiment the total mass of adhesive substance is of from 0.1mg to 3mg, preferably from 0.2mg to 2.5mg, preferably from 0.3mg to 2mg, preferably from 0.4mg to 1.8mg, preferably from 0.5mg to 1.8mg, preferably from 0.5mg to 1.5mg.

Alternatively (not shown), when the dosage form comprises one or more mechanical locking features **8,** the adhesive substance **6** may be applied in the form of one or more droplets in the location of said locking feature **8** on the surface of the cap **2,** or body **3,** or both. In this embodiment, the adhesive substance subsequently redistributes during the insertion step following capillary forces that add to the friction forces arising from sliding the cap over the body in said insertion step. This results in the advantages described above.

In an embodiment, the adhesive substance is applied as a spray (i.e. a plurality of droplets), preferably the spray being a conical spray (i.e. radiating outward in the form of a cone). In this embodiment the spray is applied on the inner surface of the cap, preferably on the mechanical locking feature such that generally substantially the entire surface of the locking feature is covered by said adhesive. In this embodiment, the droplets typically have a diameter of from 10µm to 500µm, preferably from 10µm to 300µm, more preferably from 20µm to 250µm, even more preferably from 30µm to 150µm, most preferably from 40µm to 100µm. After spraying, the cap and body may be joined in the insertion step as above described.

In any of the embodiments herein, the insertion step may comprise linear translation of the cap relative to the body in a direction substantially parallel to the centerline **Y;** or linear translation of the cap relative to the body in a direction substantially parallel to the centerline **Y** in combination with rotational translation about an axis substantially parallel to the centerline **Y.** The latter has the advantage of ensuring a skewed adhesive region **Ar** is achieved.

The abovementioned translations may be applied simultaneously from start of insertion up to when the cap and body are in a fully inserted position, or may be applied discontinuously throughout the insertion step. The advantage of the latter is that a combination of linear and skewed adhesive regions may be achieved along the length L.

### EXAMPLES AND METHODS

### Pull-apart test:

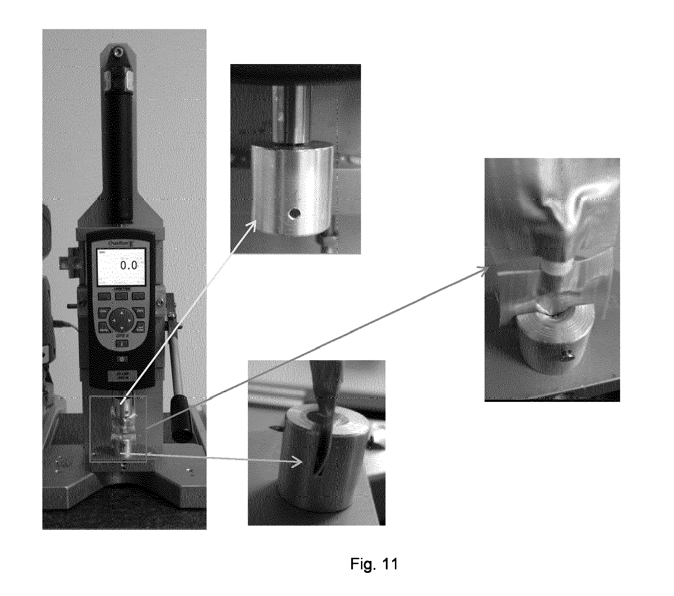

Pull-apart or opening forces as measured by Chatillon DFE II Series Digital Force Gauges from Ametek Inc. The test fixtures of the universal testing machine are modified to fixate the specimens without applying pressure force on them. Duct tape [3M 398FR] is first fixated separately around cap and body. The outer end of the duct tape is then inserted in gauge of the test fixtures and is fixated by pressing a nail through the opening of the fixtures and tape. After correct positioning of the specimen, the peak (or ultimate) tensile strength is measured in Newtons (N). The setup is shown in Fig. 11.

### Torsion test:

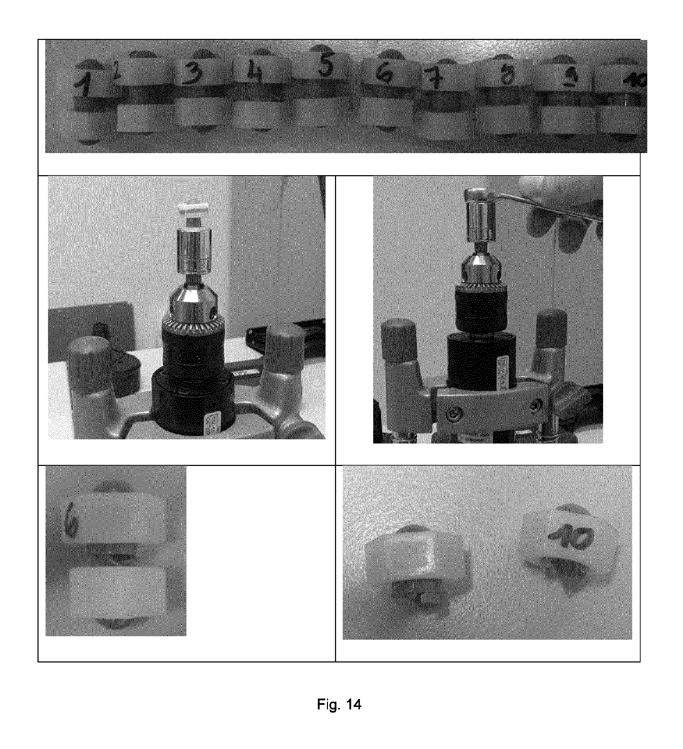

Torque measurements as measured by Chatillon® DFS II Series Digital force gauges with a chatillon® STS series torque sensor (TSD-50 OZ-IN) from Ametek Inc. Hex screw nuts of polyamide with minimal clearance are fixated around cap and body. The body with screw nut is placed into a socket mounted on the torsion gauge. A hex screw key is then used to screw the cap from the body. The setup is shown in Fig. 14.

### Example 1

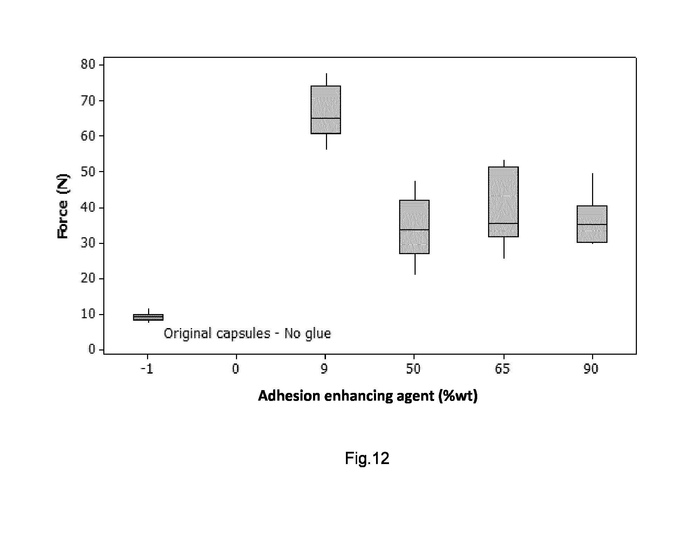

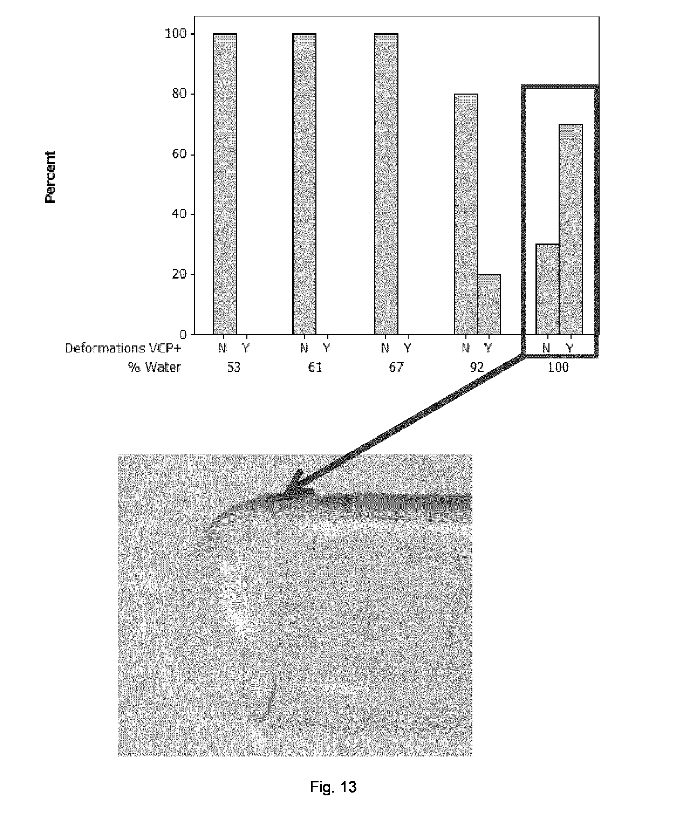

Vcaps® Plus size 0 capsules (HPMC capsules sold by Capsugel®) are used. Adhesive substances comprising water at various concentrations and sugar as adhesive enhancing agent are prepared. Two drops of adhesive substance (consisting of sucrose and water) are applied on the inner surface of the cap of each capsule (at 180° angles). Each drop is applied proximal to the rim of the cap. The cap is then inserted over the body up to a fully locked position allowing the adhesive to spread as the surfaces slide over each other, and the adhesive is allowed to dry. The capsules are then tested for pull-apart and torsion forces and deformation according to the procedures herein described. Fig. 12 is a graph showing the pull-apart force variation with %wt of adhesion enhancing agent (sucrose) in the adhesive substance and Fig.13 the % number of deformations present/not present (Y being "Yes, deformation present" and N being "No, deformation not present") with %wt of water in the adhesive substance.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm" (i.e. every value in a practical range close to 40 mm).

## Claims

1. A tamperproof dosage form (1) comprising:
a cap (2) and a body (3) each comprising an outer surface (4) and an inner surface (5), the cap (2) and body (3) being arranged to telescopically engage with each other such that an overlap region (Or) is formed between a portion of the outer surface (4) of the body (3) and a portion of the inner surface (5) of the cap (2), said overlap region (Or) comprising an overlap length (L) extending parallel to a centerline (Y) of said dosage form (1), **characterized in that** an adhesive substance (6) is comprised throughout substantially the entire overlap length (L) bonding said cap (2) to said body (3).

2. A tamperproof dosage form (1) according to claim 1 wherein the overlap region (Or) has an overlap width (W) that extends along a circumference of said dosage form (1) and the adhesive substance (6) is comprised in an adhesive region (Ar), said adhesive region (Ar) extending throughout substantially the entire overlap length (L) and at least a portion of the overlap width (W), preferably less than the total overlap width (W).

3. A tamperproof dosage form (1) according to claim 2 wherein the adhesive region (Ar) comprises an adhesive area that is at least 5%, preferably at least 10%, more preferably at least 15%, even more preferably at least 20%, of an overlap area of the overlap region (Or).

4. A tamperproof dosage form (1) according to claim 2 comprising a plurality of adhesive regions (Ar) discontinuously or continuously, preferably discontinuously, extending along the overlap width (W).

5. A tamperproof dosage form (1) according to claim 4 wherein the plurality of adhesive regions (Ar) are, preferably equally, spaced apart from one another along the overlap width (W) such that the distance between each of a neighboring adhesive region (Ar) along the overlap width (W) is less than or equal to 50%, preferably less than or equal to 25%, more preferably from greater than 20% to 50%, of the total overlap width (W).

6. A tamperproof dosage form (1) according to any of the preceding claims wherein the adhesive substance is free of organic solvents and comprises one or more adhesive enhancing agents.

7. A tamperproof dosage form (1) according to claim 6 wherein the adhesive enhancing agent is selected from the group consisting of polysaccharides, monofunctional organic acids, polyfuntional organic acids, and mixtures thereof, preferably selected from the group consisting of sucrose, fructose, lactose, maltose, cellobiose, glucose, galactose, mannose, arabinose, sorbitol, and mixtures thereof.

8. An adhesive composition for bonding one or more segments selected from a cap and body of a tamperproof dosage form according to any of the preceding claims, the adhesive composition being an aqueous composition free of organic solvents and comprising water and optionally one or more adhesive enhancing agents.

9. An adhesive composition according to claim 8 wherein water is comprised at a level of greater than 50%, preferably from 70% to less than or equal to 100%, more preferably from 80% to 95%, even more preferably from 88% to 93%, by weight of said composition.

10. An adhesive composition according to claim 8 wherein the adhesive enhancing agent is selected from the group consisting of polysaccharides, monofunctional organic acids, polyfuntional organic acids, and mixtures thereof, preferably selected from the group consisting of sucrose, fructose, lactose, maltose, cellobiose, glucose, galactose, mannose, arabinose, sorbitol, and mixtures thereof.

11. An adhesive composition according to claims 8 to 10 wherein the adhesive enhancing agent is comprised at a level of less than 50%, preferably from greater than 0% to 30%, more preferably from 5% to 20%, even more preferably from 7% to 12%, by weight of the composition.

12. An adhesive composition according to claims 8 to 11 wherein the ratio of the one or more adhesive enhancing agents to water is from 0.05:1.00 to 0.45:1.00, preferably from 0.08:1.00 to 0.13:1.00, by weight.

13. A process of making a tamperproof dosage form (1) according to claims 1 to 7, the process comprising the steps of:
providing a cap (2) and a body (3) each comprising an outer surface (4) and an inner surface (5);
applying an adhesive substance (6), preferably consisting of the adhesive composition according to claims 8 to 12, on the inner surface (5) of the cap (2) or outer surface (4) of the body (3) or both; and
telescopically inserting the cap (2) over the body (3) such that the adhesive substance is distributed throughout substantially an entire overlap length (L) once the cap (2) and body (3) reach a fully inserted position.

14. The use of an adhesive composition according to claims 8 to 12 for bonding a cap (2) and a body (3) of a dosage form to provide tamperproofness of the same.

15. A dosage form (1) according to any of the preceding claims for use in an inhalation device for dry-powder-inhalation.
